(19) Europäisches Patentamt
European Patent Office
Office européen des brevets

(11) Veröffentlichungsnummer: **0 135 854**
**B1**

(12) **EUROPÄISCHE PATENTSCHRIFT**

(45) Veröffentlichungstag der Patentschrift: **08.11.89**

(51) Int. Cl.⁴: **A 01 N 43/653**

(21) Anmeldenummer: **84110477.1**

(22) Anmeldetag: **04.09.84**

(54) **Fungizide Mittel.**

**Teilanmeldung 88121829.1 eingereicht am 04/09/84.**

(30) Priorität: **16.09.83 DE 3333411**

(43) Veröffentlichungstag der Anmeldung: **03.04.85 Patentblatt 85/14**

(45) Bekanntmachung des Hinweises auf die Patenterteilung: **08.11.89 Patentblatt 89/45**

(84) Benannte Vertragsstaaten: **BE CH DE FR GB IT LI NL**

(56) Entgegenhaltungen:
**EP-A- 0 052 424**
**FR-A- 2 516 350**

(73) Patentinhaber: **BAYER AG, D-5090 Leverkusen 1 Bayerwerk (DE)**

(72) Erfinder: **Brandes, Wilhelm, Dr., Eichendorffstrasse 3, D-5653 Leichlingen (DE)**
Erfinder: **Hänssler, Gerd, Dr., Heymannstrasse 40, D-5090 Leverkusen 1 (DE)**
Erfinder: **Reinecke, Paul, Dr., Lessingstrasse 11, D-5090 Leverkusen 3 (DE)**
Erfinder: **Scheinpflug, Hans, Dr., Am Thelenhof 15, D-5090 Leverkusen 1 (DE)**
Erfinder: **Holmwood, Graham, Dr., Krutscheider Weg 105, D-5600 Wuppertal 11 (DE)**

**EP 0 135 854 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue fungizide Wirkstoffkombinationen aus einem speziellen bekannten substituierten 1-Hydroxyethyl-triazolyl-Derivat und anderen bekannten fungiziden Wirkstoffen.

Es ist bereits allgemein bekannt, daß Mischungen enthaltend 1,2,4-Triazol-Derivate, wie z.B. das

1-(4-Chlorphenoxy)-3,3-dimethyl-1-(1,2,4-triazol-1-yl)-2-butanon,

in Kombination mit anderen bekannten Fungiziden eine beachtlich höhere Wirkung als die Einzelkomponenten aufweisen (vgl. z.B. die DE-A 2 552 967). Die Wirksamkeit dieser Wirkstoffmischungen ist jedoch nicht auf allen Anwendungsgebieten voll befriedigend.

Es wurde gefunden, daß neue Wirkstoffkombinationen aus

– dem substituierten 1-Hydroxyethyl-triazolyl-Derivat der Formel

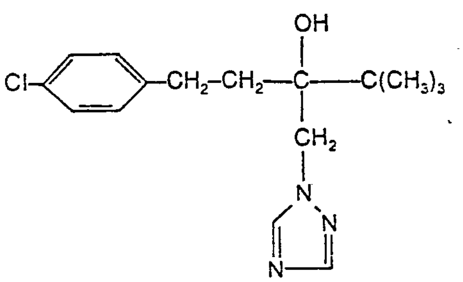

(I)

und

– einem Polyhalogenalkylthio-Derivat der Formel

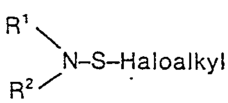

(II)

in welcher

(IIa) $R^1 = (CH_3)_2N-SO_2-$, $R^2 =$ 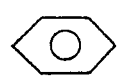 ,

Haloalkyl $= -CCl_2F$

(IIb) $R^1$ und $R^2 =$ 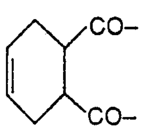 ,

Haloalkyl $= -CCl_3$

(IIc) $R^1$ und $R^2 =$ 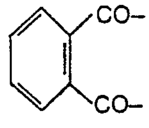 ,

Haloalkyl $= -CCl_3$

(IId) $R^1$ und $R^2 =$ 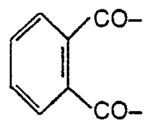 ,

Haloalkyl $= -CCl_2-CHCl_2$

bedeuten,

wobei in der Wirkstoffkombination das Gewichtsverhältnis von substituiertem 1-Hydroxyethyl-triazolyl-Derivat der Formel (I) zu Polyhalogenalkylthio-Derivat der Formel (II) zwischen 1:0,1 und 1:500 liegt, eine besonders hohe fungizide Wirksamkeit aufweisen.

Überraschenderweise ist die fungizide Wirkung der erfindungsgemäßen Wirkstoffkombinationen wesentlich höher als die Wirkung der Einzelkomponenten und gegebenenfalls auch als die Summe der Einzelkomponenten (synergistischer Effekt). Die Auffindung dieser Kombinationen aus der speziellen Verbindung der Formel (I) und den Wirkstoffen der oben angegebenen Formel (II) stellt somit eine wertvolle Bereicherung der Technik dar.

Das für die erfindungsgemäße Kombination speziell zu verwendende substituierte 1-Hydroxyethyl-triazolyl-Derivat ist durch die obige Formel (I) eindeutig definiert.

Die genannte Verbindung bzw. ihre Herstellung sind bereits beschrieben (vgl. EP-A 0 040 345 und EP-A 0 052 424).

Die als Mischungskomponenten zu verwendenden Verbindungen der Formel (II) sind bekannt aus R. Wegler, «Chemie der Pflanzenschutz- und Schädlingsbekämpfungsmittel», Band 2, Seiten 95, 108, 109 und 110, Springer Verlag Berlin / Heidelberg / New York, 1970.

Zu einer Wirkstoffkombination aus dem substituierten 1-Hydroxyethyl-triazol-Derivat der Formel (I) und den Wirkstoffen der Formel (II) können noch weitere Wirkstoffe (z.B. als Drittkomponente) hinzukommen.

Die Gewichtsverhältnisse der Wirkstoffe in den Wirkstoffkombinationen können in relativ großen Bereichen schwanken. Im allgemeinen entfallen auf 1 Gew.-Teil an Verbindung der Formel (I) 0,1 bis 500 Gew.-Teile Wirkstoff der Formel (II) vorzugsweise 0,2 bis 200 Gew.-Teile an Wirkstoff der Formel (II), besonders bevorzugt 0,5 bis 50 Gew.-Teile.

Die erfindungsgemäßen Wirkstoffkombinationen weisen eine starke mikrobizide Wirkung auf und können zur Bekämpfung von unerwünschten Mikroorganismen praktisch eingesetzt werden; sie sind für den Gebrauch als Pflanzenschutzmittel geeignet.

Fungizide Mittel im Pflanzenschutz werden eingesetzt zur Bekämpfung von Plasmodiophoromycetes, Oomyctes, Chytridiomycetes, Zygomycetes, Ascomycetes, Basidiomycetes, Deuteromycetes.

Die gute Pflanzenverträglichkeit der Wirkstoffkombinationen in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffkombinationen haben ein sehr breites Wirkungsspektrum und

können angewandt werden gegen parasitäre Pilze, die oberirdische Pflanzenteile befallen oder die Pflanzen vom Boden her angreifen, sowie samenübertragbare Krankheitserreger. Besonders praktische Bedeutung haben solche Wirkstoffkombinationen als Saatgutbeizmittel gegen phytopathogene Pilze, die mit dem Saatgut übertragen werden oder im Boden vorkommen und von dort die Kulturpflanzen befallen. Dabei handelt es sich um Keimlingskrankheiten, Wurzelfäulen, Stengel-, Halm-, Blatt-, Blüten-, Frucht- und Samenkrankheiten, die insbesondere durch Tilletia-Urocystis-, Ustilago-, Septoria-, Typhula-, Rhynchosporium-, Helminthosporium- und Fusarium-Arten hervorgerufen werden. Durch die systemische Wirkung des einen Mischungspartners werden die Pflanzen auch oft längere Zeit nach der Beizung noch vor Krankheitserregern geschützt, die verschiedene Teile des Sprosses angreifen können, z.B. echte Mehltaupilze und Rostpilze. Die Wirkstoffkombinationen können daneben auch als Bodenbehandlungsmittel gegen phytopathogene Pilze eingesetzt werden und wirken gegen Wurzelfäulen und Tracheomykosen, die z.B. durch Krankheitserreger der Gattungen Pythium, Verticillium, Phialophora, Rhizoctonia, Fusarium und Thielaviopsis verursacht werden.

Die erfindungsgemäsen Wirkstoffkombinationen zeigen aber auch hervorragende Wirkung bei direkter Applikation auf die oberirdischen Pflanzenteile gegen Krankheitserregner auf verschiedenen Kulturpflanzen, wie echte Mehltaupilze (Erysiphe-, Uncinula-, Sphaerotheca-, Podosphaera-Arten, Leveillula taurica), Rostpilze, Venturia-Arten, Cercospora-Arten, Alternaria-Arten, Botrytis-Arten, Phytophthora-Arten, Peronospora-Arten, Fusarium-Arten, Pyrenophora-Arten, Cochliobolus-Arten, Septoria-Arten, Pseudocercosporella herpotrichoides, Pyricularia oryzae, Pellicularia sasakii.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Zur Erläuterung dienen die nachfolgenden Anwendungsbeispiele.

Beispiel A

Phytophthora-Test (Tomate)/protektiv

Lösungsmittel: 4,7 Gewichtsteile Aceton

Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100% relativer Luftfeuchtigkeit und ca. 20 °C aufgestellt.

3 Tage nach der Inokulation erfolgt die Auswertung.

Tabelle A

Phytophthora-Test (Tomate)/protektiv

| Wirkstoff | Befall in % bei einer Wirkstoffkonzentration von | |
|---|---|---|
| (IIa) (bekannt) (DICHLOFLUANID) | 0,00025 | 54 |
| (I) (bekannt) | 0,000005 | 83 |
| Mischung aus (I) und (IIa) (Mischungsverhältnis 1:50) | 0,000005 + 0,00025 | 37 |

Beispiel B

Leptosphaeria nodorum-Test (Weizen)/protektiv

Lösungsmittel: 100 Gewichtsteile Dimethylformamid

Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20 °C und 100% relativer Luftfeuchtigkeit in einer Inkubationskabine.

Die Pflanzen werden in einem Gewächshaus bei einer Temperatur von ca. 15 °C und einer relativen Luftfeuchtigkeit von ca. 80% aufgestellt.

10 Tage nach der Inokulation erfolgt die Auswertung.

Um Synergismus zwischen den in diesem Versuch verwendeten Wirkstoffen aufzuzeigen, wurden die Resultate nach der von R.S. Colby beschriebenen Methode (Calculating Synergistic and Antagonistic Responses of Herbicides Combinations; Weeds 15, 20–22, 1967) ausgewertet. Der erwartete Befall in % der unbehandelten Kontrolle wurde gemäß der Gleichung

$$E = \frac{X \cdot Y}{100}$$

berechnet. Dabei bedeuten X bzw. Y den Krankheitsbefall – ausgedrückt in % der unbehandelten Kontrolle –, den die beiden Präparate bei einer getrennten Anwendung zulassen. Ein synergistischer Effekt liegt dann vor, wenn die fungizide Wirkung der Wirkstoffkombination größer ist als die der einzeln applizierten Wirkstoffe. In diesem Fall muß der tatsächlich beobachtete Befall geringer sein als der aus der oben angeführten Formel errechnete Wert für den erwarteten Befall (E).

Tabelle B
Leptosphaeria nodorum-Test (Weizen)/protektiv

| Wirkstoff | Wirkstoffkonzentration in der Spritzbrühe in Gew.-% | Krankheitsbefall in % der unbehandelten Kontrolle | |
|---|---|---|---|
| (I) (bekannt) | 0,025 | 100 | |
| CAPTAFOL (IId) (bekannt) | 0,01 | 82,5 | |
| | | beobachteter Befall nach Anwendung der Mischung in % der unbehandelten Kontrolle | erwarteter Befall (E) nach Anwendung der Mischung |
| Mischung aus I und IId (Mischungsverhältnis 2,5:1) | 0,025 +0,01 | 50,0 | 82,5 |

Herstellungsbeispiele:

Beispiel 1

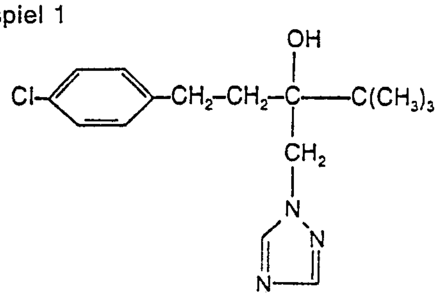

Eine Lösung von 17,9 g (0,075 Mol) 2-(4-Chlorphenylethyl)-2-tert.-butyl-oxiran und 6,9 g (0,1 Mol) 1,2,4-Triazol in 30 ml Ethanol wird 20 Stunden bei 150 °C im Bombenrohr erhitzt. Man läßt abkühlen und engt die Reaktionslösung ein. Der Rückstand wird in Ether gelöst, dreimal mit Wasser und einmal mit Natriumchloridlösung gewaschen, über Natriumsulfat getrocknet und eingeengt. Der Rückstand wird über eine Kieselgelsäure chromatographiert (Laufmittel : Dichlormethan/Essigester = 1:1).

Man erhält 12,3 g (53,2% der Theorie)
1-(4-Chlorphenyl)-4,4-dimethyl-3-(1,2,4-triazol-1-yl-methyl)-pentan-3-ol
als zähflüssiges Öl, das sich aus Acetonitril umkristallisieren läßt (Schmelzpunkt 102 °C bis 104 °C).

**Patentansprüche**

1. Fungizides Mittel, gekennzeichnet durch einen Gehalt an einer Wirkstoffkombination aus

- dem substituierten 1-Hydroxyethyl-triazolyl-Derivat der Formel

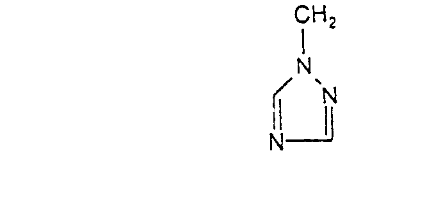

(I)

und

- einem Polyhalogenalkylthio-Derivat der Formel

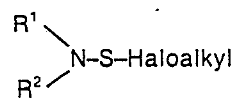

(II)

in welcher

(IIa) $R^1$ = $(CH_3)_2N-SO_2-$, $R^2$ = 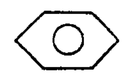,

Haloalkyl = $-CCl_2F$

(IIb) $R^1$ und $R^2$ = 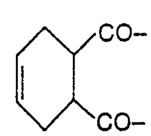,

Haloalkyl = $-CCl_3$

(IIc) $R^1$ und $R^2$ = 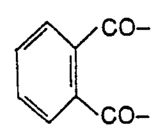,

Haloalkyl = $-CCl_3$

(IId) $R^1$ und $R^2$ = 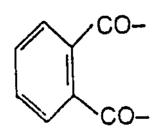,

Haloalkyl = $-CCl_2-CHCl_2$

bedeuten,

wobei in der Wirkstoffkombination das Gewichtsverhältnis von substituiertem 1-Hydroxyethyl-triazolyl-Derivat der Formel (I) zu Polyhalogenalkylthio-Derivat der Formel (II) zwischen 1:0,1 und 1:500 liegt.

2. Fungizides Mittel gemäß Anspruch 1, dadurch gekennzeichnet, daß das Gewichtsverhältnis zwischen 1:0,2 und 1:200 liegt.

3. Fungizides Mittel gemäß Anspruch 1, dadurch gekennzeichnet, daß das Gewichtsverhältnis zwischen 1:0,5 und 1:50 liegt.

4. Verfahren zur Bekämpfung von Pilzen, dadurch gekennzeichnet, daß man eine Wirkstoffkombination gemäß Anspruch 1 auf Pilze oder deren Lebensraum einwirken läßt.

5. Verwendung von Wirkstoffkombinationen gemäß Anspruch 1 zur Bekämpfung von Pilzen.

6. Verfahren zur Herstellung von fungiziden Mitteln, dadurch gekennzeichnet, daß man eine Wirkstoffkombination gemäß Anspruch 1 mit Streckmitteln und/oder oberflächenaktiven Mitteln vermischt.

**Claims**

1. Fungicidal agent, characterized in that it contains an active compound combination of
– the substituted 1-hydroxyethyl-triazolyl derivative of the formula

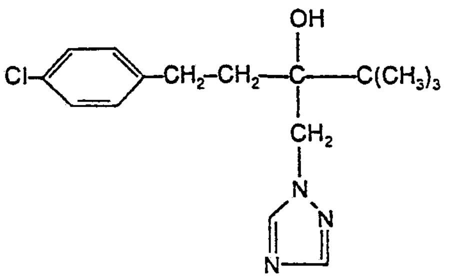 (I)

and
– a polyhalogenoalkylthio derivative of the formula

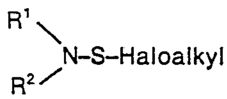 (II)

in which
(IIa) $R^1$ = $(CH_3)_2N-SO_2-$, $R^2$= 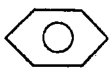 ,

haloalkyl = $-CCl_2F$

(IIb) $R^1$ and $R^2$ = 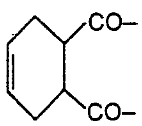 ,

haloalkyl = $-CCl_3$

(IIc) $R^1$ and $R^2$ = 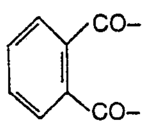 ,

haloalkyl = $-CCl_3$

(IId) $R^1$ and $R^2$ = 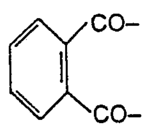 ,

haloalkyl = $-CCl_2-CHCl_2$

wherein the active compound combination the weight ratio of substituted 1-hydroxyethyl-triazolyl derivative of the formula (I) to polyhalogenoalkylthio derivative of the formula (II) is between 1:0.1 and 1:500.

2. Fungicidal agent according to Claim 1, characterized in that the weight ratio is between 1:0.2 and 1:200.

3. Fungicidal agent according to Claim 1, characterized in that the weight ratio is between 1:0.5 and 1:50.

4. Method of combating fungi, characterized in that an active compound combination according to Claim 1 is allowed to act on fungi or their environment.

5. Use of active compound combinations according to Claim 1 for combating fungi.

6. Process for the preparation of fungicidal agents, characterized in that an active compound combination according to Claim 1 is mixed with extenders and/or surface-active agents.

**Revendications**

1. Composition fongicide, caractérisée par une teneur en une association de substances actives constituée
– du dérivé 1-hydroxyéthyl-triazolylique substitué de formule

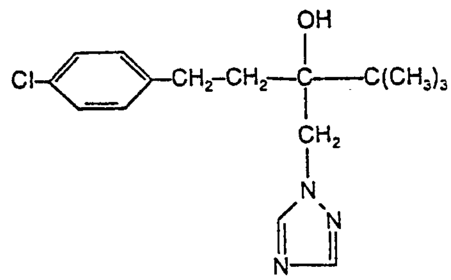 (I)

et
– d'un dérivé polyhalogénalkylthio de formule

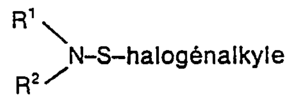 (II)

dans laquelle
(IIa) $R^1$ = $(CH_3)_2N-SO_2-$, $R^2$= 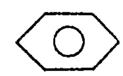 ,

halogénalkyle = $CCl_2F$

(IIb) $R^1$ et $R^2$ 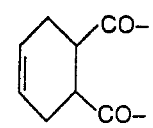 ,

halogénalkyle = $CCl_3$

(IIc) $R^1$ et $R^2$ = 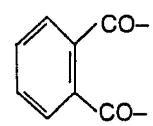 ,

halogénalkyle = $CCl_3$

(IId) $R^1$ et $R^2$ = 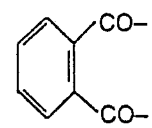 ,

halogénalkyle = $-CCl_2-CHCl_2$

le rapport du poids de dérivé 1-hydroxyéthyl-triazolylique substitué de formule (I) au poids de dérivé polyhalogénalkylthio de formule (II) dans l'association de substances actives ayant une valeur comprise entre 1:0,1 et 1:500.

2. Composition fongicide suivant la revendication 1, caractérisée en ce que le rapport des poids se situe entre 1:0,2 et 1:200.

3. Composition fongicide suivant la revendication 1, caractérisée en ce que le rapport des poids se situe entre 1:0,5 et 1:50.

4. Procédé pour combattre des champignons, caractérisé en ce qu'on fait agir une association

de substances actives suivant la revendication 1 sur des champignons ou sur leur milieu.

5. Utilisation d'associations de substances actives suivant la revendication 1 pour combattre des champignons.

6. Procédé de préparation de compositions fongicides, caractérisé en ce qu'on mélange une association de substances actives suivant la revendication 1 avec des diluants et/ou des agents tensio-actifs.